(19) Europäisches Patentamt
European Patent Office
Office européen des brevets

(11) **EP 2 858 744 B1**

(12) **FASCICULE DE BREVET EUROPEEN**

(45) Date de publication et mention de la délivrance du brevet:
**14.08.2019 Bulletin 2019/33**

(21) Numéro de dépôt: **13733361.3**

(22) Date de dépôt: **06.06.2013**

(51) Int Cl.:
**B01J 8/02** *(2006.01)*      **B01J 8/06** *(2006.01)*
**B01J 19/24** *(2006.01)*

(86) Numéro de dépôt international:
**PCT/FR2013/051296**

(87) Numéro de publication internationale:
**WO 2013/182818 (12.12.2013 Gazette 2013/50)**

(54) **REACTION CATALYTIQUE AVEC REGENERATION EN FLUX INVERSE**

KATALYTISCHE REAKTION MIT RÜCKSTROMREGENERATION

CATALYTIC REACTION WITH REVERSE-FLOW REGENERATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.06.2012 FR 1255368**

(43) Date de publication de la demande:
**15.04.2015 Bulletin 2015/16**

(73) Titulaire: **Arkema France**
**92700 Colombes (FR)**

(72) Inventeur: **DUBOIS, Jean-Luc**
**F-69390 Millery (FR)**

(74) Mandataire: **Albani, Dalila**
**ARKEMA France**
**Département Propriété Industrielle**
**420, rue d'Estienne d'Orves**
**92705 Colombes Cedex (FR)**

(56) Documents cités:
**EP-A1- 0 154 674      DE-A1- 10 239 547**
**US-A- 4 461 745**

• **KOLIUS G ET AL: "AUTOTHERMAL FIXED-BED REACTOR CONCEPTS", CHEMICAL ENGINEERING SCIENCE, OXFORD, GB, vol. 55, 1 janvier 2000 (2000-01-01), pages 5945-5967, XP002265573, ISSN: 0009-2509, DOI: 10.1016/S0009-2509(00)00183-4**

EP 2 858 744 B1

**Description**

DOMAINE DE L'INVENTION

La présente invention concerne un procédé de réaction chimique comprenant des phases alternées de réaction et de régénération. L'invention concerne également l'utilisation d'un réacteur chimique adapté à la mise en oeuvre de ce procédé.

ARRIERE-PLAN TECHNIQUE

On connaît la mise en oeuvre de réactions chimiques, notamment impliquant une catalyse hétérogène, dans des réacteurs à lit fixe. Dans des procédés de ce type, le contrôle de la chaleur absorbée ou émise représente un aspect critique.

Par exemple, dans les réactions exothermiques telles que les réactions de combustion, un front chaud a tendance à se former dans le réacteur lorsque la réaction démarre, et ce front chaud a tendance par la suite à se déplacer dans le lit du réacteur selon la direction du flux, jusqu'à éventuellement présenter un risque de sortie du lit.

Afin de résoudre ce problème, des réacteurs à flux inversé ont été mis au point. Le principe de ces systèmes repose sur une inversion du flux réactionnel permettant de maintenir le front chaud dans le lit du réacteur au cours de la réaction.

Les documents WO 02/051965 et US 5,710,356 fournissent des exemples de tels systèmes symétriques, dans lesquels le même flux passe à travers le réacteur alternativement dans un sens et dans le sens opposé.

Le document US 7,763,174 décrit une variante dans laquelle des lits adsorbants sont placés de chaque côté du lit de catalyseur.

Toujours dans un but d'optimisation des transferts de chaleur, des procédés de réaction à flux inversé asymétriques ont également été proposés, afin de permettre le couplage thermique d'une réaction exothermique et d'une réaction endothermique. Par exemple, un premier sens d'écoulement peut être utilisé pour réaliser la combustion de méthane (injection de méthane et d'air), tandis que le sens inverse d'écoulement peut être utilisé pour réaliser le reformage du méthane (injection de méthane et d'eau). La première réaction est destinée à chauffer le lit de catalyseur jusqu'à un niveau suffisant pour permettre la deuxième réaction, endothermique, de reformage du méthane. Le document US 2004/0170559 fournit une illustration d'un tel procédé asymétrique.

Dans les systèmes de réacteurs à flux inversé décrits ci-dessus, aucune mesure spécifique n'est prévue pour la régénération du catalyseur, une fois celui-ci désactivé.

Le document DE 10239547 illustre un autre exemple d'un couplage thermique d'une réaction endothermique et d'une réaction exothermique, cette fois en combinant le reformage de méthane avec la régénération du catalyseur. Selon ce procédé, le reformage de méthane à la vapeur d'eau est réalisé à 400°K dans un réacteur comprenant des distributeurs pour l'injection d'un flux supplémentaire amené par des conduites d'alimentation intégrées au réacteur. Lors de la régénération, la direction d'écoulement est inversée et les distributeurs internes sont alimentés par un combustible pour atteindre une température de régénération de 1000°K.

Le procédé mis en oeuvre dans le document US 4,461,745 consiste à effectuer de façon simultanée une réaction et une régénération, chacune dans une moitié d'une enceinte réactionnelle, puis à inverser ces 2 phases, le flux issu de l'étape de régénération étant toujours mélangé dans l'enceinte réactionnelle avec le flux réactionnel pour alimenter la zone en phase de réaction.

Dans le cadre de réactions catalytiques de fluoration ou de production de composés acryliques mises en oeuvre en lit fixe, le catalyseur se désactive notamment par accumulation de coke sur celui-ci. La régénération du catalyseur peut être effectuée par combustion du coke, en injectant un flux riche en oxygène ou en air dans le réacteur.

Cependant, la proportion de coke dans le réacteur n'est pas homogène : il y a en principe davantage de coke accumulé vers l'entrée du réacteur que vers la sortie de celui-ci. Cela pose un problème de contrôle de la combustion. Par exemple, un front chaud peut apparaître, entraînant un différentiel très élevé de température, qui est susceptible d'altérer le catalyseur.

Il existe donc un besoin de parvenir à un meilleur contrôle de la phase de régénération de ce type de procédés, notamment en évitant les risques de dégradation du catalyseur.

RESUME DE L'INVENTION

L'invention concerne en premier lieu un procédé de réaction chimique mis en oeuvre dans une enceinte réactionnelle contenant un lit de catalyseur,
l'enceinte réactionnelle comprenant une première extrémité et une deuxième extrémité opposée à la première extrémité,
le procédé comprenant de manière alternée :

- une phase de réaction, comprenant le passage d'un flux réactionnel dans l'enceinte réactionnelle de sa première extrémité vers sa deuxième extrémité ; et
- une phase de régénération du catalyseur, comprenant le passage d'un flux de régénération dans l'enceinte réactionnelle de sa deuxième extrémité vers sa première extrémité ;

caractérisé en ce que l'enceinte réactionnelle est constituée d'une pluralité de compartiments réactionnels distincts contenant un lit de catalyseur et comprenant un système d'échange thermique entre eux Selon l'invention, les échanges thermiques sont assurés efficacement.

La phase de réaction et la phase de régénération sont deux phases distinctes réalisées sur un même ensemble de lits de catalyseur de façon successive et en alternance.

Selon un mode de réalisation, le flux de régénération comprend de l'oxygène, et la phase de régénération du catalyseur comprend la combustion de coke déposé sur le catalyseur.

Selon l'invention, le procédé de réaction chimique est :

- un procédé de déshydratation du glycérol en acroléine ; ou
- un procédé de déshydratation de l'acide lactique ou du lactate d'ammonium en acide acrylique ; ou
- un procédé de déshydratation de l'acide 3-hydroxypropionique ou de son sel d'ammonium en acide acrylique ; ou
- un procédé de déshydratation de l'acide 2-hydroxyisobutyrique ou de l'acide 3-hydroxyisobutyrique ou de leurs sels d'ammonium correspondants en acide méthacrylique ; ou
- un procédé de conversion d'un composé chloré en un composé fluoré, de préférence un procédé de préparation d'une hydrofluorooléfine ou d'un hydrofluorocarbure, notamment un procédé de préparation d'un fluoropropène, et de manière tout particulièrement préférée un procédé de préparation du 2,3,3,3-tétrafluoropropène ; ou
- un procédé d'oxydation sélective telle que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique ;
- l'oxydation du benzène, du butène, du butanol ou du butane en anhydride maléique ; l'oxydation du propylène en acroléine ; l'oxydation de l'isobutène ou du tertiobutanol en méthacroléine.

Selon un mode de réalisation, le procédé est un procédé de réaction exothermique.

Selon un mode de réalisation, la phase de réaction présente une durée supérieure ou égale à 15 minutes, de préférence supérieure ou égale à 1 heure ou supérieure ou égale à 15 heures; et / ou la phase de régénération présente une durée supérieure ou égale à 15 minutes, de préférence supérieure ou égale à 5 heures.

Selon un mode de réalisation, la phase de réaction et la phase de régénération ont des durées différentes, en particulier la durée de la phase de régénération est inférieure à la durée de la phase de réaction.

Selon un mode de réalisation, la phase de réaction et la phase de régénération ont des durées prédéterminées ; ou la durée de la phase de réaction et / ou la durée de la phase de régénération dépendent de la mesure d'un paramètre tel qu'une température dans le réacteur ou la teneur d'un composé dans un flux sortant du réacteur.

L'invention concerne également l'utilisation d'un réacteur adapté à la mise en oeuvre d'un procédé de réaction chimique comprenant de manière alternée une phase de réaction et une phase de régénération tel que défini précédemment. Ce réacteur est un réacteur chimique comprenant :

- une enceinte réactionnelle contenant un lit de catalyseur, l'enceinte réactionnelle comprenant une première extrémité et une deuxième extrémité opposée à la première extrémité, ainsi qu'une pluralité de compartiments réactionnels distincts contenant un lit de catalyseur avec un système d'échange thermique entre eux ;
- une conduite d'amenée de flux réactionnel connectée en entrée de l'enceinte réactionnelle à sa première extrémité ;
- une conduite de soutirage de flux réactionnel connectée en sortie de l'enceinte réactionnelle à sa deuxième extrémité ;
- une conduite d'amenée de flux de régénération connectée en entrée de l'enceinte réactionnelle à sa deuxième extrémité ;
- une conduite de soutirage de flux de régénération connectée en sortie de l'enceinte réactionnelle à sa première extrémité ;
- des moyens de commutation adaptés pour permettre alternativement le passage d'un flux réactionnel dans l'enceinte réactionnelle depuis la conduite d'amenée de flux réactionnel jusqu'à la conduite de soutirage de flux réactionnel, et le passage d'un flux de régénération depuis la conduite d'amenée de flux de régénération jusqu'à la conduite de soutirage de flux de régénération,

le réacteur étant de préférence un réacteur à plaques ou un réacteur multitubulaire.

La présente invention permet de surmonter les inconvénients de l'état de la technique. Elle permet plus particulièrement de parvenir à un meilleur contrôle de la phase de régénération (*in situ*) du catalyseur, dans le cadre des réactions chimiques sur lit fixe, notamment en évitant les risques de dégradation du catalyseur.

Cela est accompli grâce à l'inversion du sens du flux dans le réacteur entre la phase de réaction d'une part et la phase de régénération d'autre part.

Selon certains modes de réalisation particuliers, l'invention présente également une ou de préférence plusieurs des caractéristiques avantageuses énumérées ci-dessous :

- L'inversion du sens du flux lors de la régénération permet de démarrer la combustion du coke dans la zone du réacteur qui est relativement la moins chargée en coke. De la sorte, on évite un gradient de température trop élevé risquant de modifier la structure cristalline du catalyseur et ainsi de le dégrader.
- Une régénération sans inversion du sens du flux conduit à une combustion préférentielle du coke en entrée (du fait de l'apparition du front chaud en entrée, et de la moindre concentration d'oxygène en sortie). Par conséquent, l'inversion du sens du flux lors de la régénération prévue par l'invention permet de réduire la quantité de coke dans le réacteur d'une manière plus homogène le long de celui-ci. Une telle réduction plus homogène de la quantité de coke peut d'ailleurs conduire à conserver après la régénération une proportion de coke plus importante en entrée du réacteur qu'en sortie (par rapport au sens du flux réactionnel), tout comme avant la régénération. La conservation de ce différentiel de coke entre l'entrée et la sortie permet d'éviter d'avoir un volume de lit catalytique excessivement réactif en entrée qui peut conduire à des réactions parasites. En d'autres termes, l'invention permet une meilleure maîtrise de l'activité du catalyseur.
- Lors de la phase de réaction tout comme lors de la phase de régénération, il peut y avoir une sublimation partielle de métaux du lit catalytique, et dépôt de ceux-ci en aval. L'inversion du sens du flux entre la réaction et la régénération permet de maintenir ces métaux dans le lit catalytique et d'éviter leur fuite hors du réacteur.
- Une régénération sans inversion du sens du flux peut conduire à une entrée de réacteur plus froide que la sortie à la fin de l'étape de régénération (du fait du déplacement du front chaud lors de la régénération). Il est donc nécessaire de réchauffer le lit catalytique pour redémarrer la réaction. Une telle mesure n'est pas nécessaire, ou est moins nécessaire, si l'on inverse le sens du flux lors de la régénération, puisque c'est alors l'entrée du réacteur qui est plus chaude que la sortie en fin de phase de régénération.

BREVE DESCRIPTION DES FIGURES

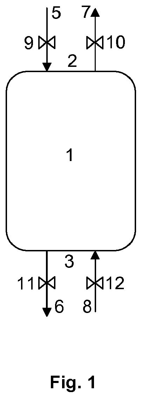

La **figure 1** représente de manière schématique un réacteur chimique selon l'invention.

DESCRIPTION DE MODES DE REALISATION DE L'INVENTION

L'invention est maintenant décrite plus en détail et de façon non limitative dans la description qui suit.

En faisant référence à la **figure 1,** de manière générale, le réacteur selon l'invention comprend une enceinte réactionnelle 1, qui comporte une première extrémité 2 et une deuxième extrémité 3 opposée à la première. Un lit fixe de catalyseur est disposé dans l'enceinte réactionnelle 1.

Par exemple, le réacteur peut être de type vertical, auquel cas la première extrémité 2 peut être située en haut de l'enceinte réactionnelle 1 et la deuxième extrémité 3 être située en bas de l'enceinte réactionnelle 1, comme illustré, mais la configuration inverse est également possible. Le réacteur peut aussi être de type horizontal.

Le réacteur comporte une conduite d'amenée de flux réactionnel 5, qui est connectée en entrée de l'enceinte réactionnelle 1 à sa première extrémité 2, et une conduite de soutirage de flux réactionnel 6 connectée en sortie de l'enceinte réactionnelle 1 à sa deuxième extrémité 3.

Le réacteur comporte par ailleurs une conduite d'amenée de flux de régénération 8 connectée en entrée de l'enceinte réactionnelle 1 à sa deuxième extrémité 3, et une conduite de soutirage de flux de régénération 7 connectée en sortie de l'enceinte réactionnelle 1 à sa première extrémité 2.

On peut éventuellement prévoir, pour chacune des conduites ci-dessus, une conduite unique, mais également plusieurs conduites en parallèle. On prend dans la suite l'exemple de conduites uniques.

L'enceinte réactionnelle 1 peut être un compartiment réactionnel unique, par exemple de type cylindrique à section circulaire (réacteur adiabatique). Mais de préférence selon l'invention, l'enceinte réactionnelle 1 comporte une pluralité de compartiments réactionnels distincts, chacun contenant son propre lit fixe de catalyseur. Dans ce cas, les compartiments réactionnels sont disposés en parallèle, c'est-à-dire que des systèmes de distribution (non représentés) permettent de répartir un flux de la conduite d'amenée de flux réactionnel 5 vers l'ensemble des compartiments réactionnels, de répartir un flux de la conduite d'amenée de flux de régénération 8 vers l'ensemble des compartiments réactionnels, de collecter un flux de l'ensemble des compartiments réactionnels vers la conduite de soutirage de flux réactionnel 6, et de collecter un flux de l'ensemble des compartiments réactionnels vers la conduite de soutirage de flux de régénération 7.

Les compartiments réactionnels peuvent par exemple être des compartiments tubulaires (cylindriques à section circulaire), dans le cas d'un réacteur multitubulaire. Ils peuvent aussi être des compartiments parallélépipédiques, dans le cas d'un réacteur à plaques.

Un système d'échange thermique (connu en soi) est prévu entre les compartiments réactionnels pour permettre les échanges de chaleur.

Chaque compartiment réactionnel comporte un lit de catalyseur. Le catalyseur peut être sous forme de particules solides (billes, grains ou poudre) ou sous forme de monolithe poreux ou de blocs de monolithe poreux.

Le lit de catalyseur peut également comprendre deux parties, chacune comportant un catalyseur différent. Dans ce cas, il peut être avantageux de prévoir un réacteur vertical, le catalyseur à plus faible durée de vie étant disposé au-dessus du catalyseur à durée de vie plus élevée, afin de permettre son remplacement plus facile. Dans ce cas, la réaction est mise en oeuvre par injection d'un flux du bas vers le haut.

Le réacteur fonctionne en alternance selon deux phases, à savoir une phase de réaction, au cours de laquelle la réaction chimique souhaitée a lieu dans l'enceinte réactionnelle 1, et au cours de laquelle le catalyseur a tendance à se désactiver progressivement ; et un mode de régénération, au cours duquel le catalyseur au moins partiellement désactivé est régénéré.

Dans le cadre de la présente demande, l'expression « flux réactionnel » est comprise au sens large et désigne un flux traversant l'enceinte réactionnelle 1 de la première extrémité 2 vers la deuxième extrémité 3 et permettant la mise en oeuvre de la réaction, étant entendu que la composition du flux réactionnel varie entre l'entrée et la sortie du réacteur.

De même, l'expression « flux de régénération » désigne un flux traversant l'enceinte réactionnelle 1 de la deuxième extrémité 3 vers la première extrémité 2 et permettant la mise en oeuvre de la régénération du catalyseur, étant entendu que la composition du flux de régénération varie entre l'entrée et la sortie du réacteur.

En phase de réaction, c'est le flux réactionnel qui traverse l'enceinte réactionnelle 1. En entrée, ce flux réactionnel comprend les réactifs nécessaires à la mise en oeuvre de la réaction, et en sortie il comprend au moins en partie les produits de la réaction.

En phase de régénération, c'est le flux de régénération qui traverse l'enceinte réactionnelle 1. En entrée, ce flux de régénération comprend un ou plusieurs composés aptes à régénérer le catalyseur, et en sortie il comprend les résidus de la régénération.

Chacun des flux peut être de type liquide ou gazeux ou mixte.

La conduite d'amenée de flux réactionnel 5 est avantageusement connectée à un réservoir de flux réactionnel, et la conduite d'amenée de flux de régénération 8 est avantageusement connectée à un réservoir de flux de régénération.

Un système de vannes 9, 10, 11, 12 permet de fermer et d'ouvrir chacune des conduites mentionnées ci-dessus, de sorte à faire fonctionner le réacteur alternativement en phase de réaction et en phase de régénération, grâce à des moyens de commutation non représentés.

Les phases de réaction et de régénération peuvent avoir des durées prédéterminées, en fonction de profils connus de désactivation et de régénération du catalyseur. Il s'agit de manière préférée de durées différentes, à savoir une durée de régénération plus courte que la durée de la phase de réaction. Alternativement, le passage de la phase de réaction à la phase de régénération et réciproquement peut être effectué en fonction d'un paramètre mesuré, tel qu'un profil de température, ou la concentration d'une substance dans le flux de sortie (par exemple la concentration en un produit de réaction dans le flux réactionnel en sortie, ou la concentration d'oxygène ou de monoxyde de carbone ou de dioxyde de carbone dans le flux de régénération en sortie).

Selon un mode de réalisation, la durée de la phase de réaction est supérieure ou égale à 15 minutes, de préférence supérieure ou égale à 1 heure, ou à 3 heures, ou à 5 heures, ou à 10 heures, ou à 15 heures.

Selon un mode de réalisation, la durée de la phase de régénération est supérieure ou égale à 15 minutes, de préférence supérieure ou égale à 1 heure, ou à 3 heures, ou à 5 heures.

Selon certains modes de réalisation, la durée de la phase de réaction est inférieure ou égale à 200 heures, ou inférieure ou égale à 100 heures, ou inférieure ou égale à 50 heures, ou inférieure ou égale à 25 heures.

Selon certains modes de réalisation, la durée de la phase de régénération est inférieure ou égale à 200 heures, ou inférieure ou égale à 100 heures, ou inférieure ou égale à 50 heures, ou inférieure ou égale à 25 heures, ou inférieure ou égale à 15 heures.

Selon un mode de réalisation, la régénération du catalyseur n'est que partielle lors de la phase de régénération (c'est-à-dire qu'on interrompt la phase de régénération avant que la régénération du catalyseur soit totale ou quasi-totale), il reste ainsi par exemple une partie du coke dans le réacteur en entrée de zone réactionnelle, ce qui permet d'optimiser la durée d'utilisation du réacteur en phase de réaction, de minimiser la durée en phase de régénération, et d'optimiser la productivité globale.

L'invention est par exemple mise en oeuvre pour la production d'acroléine et/ou d'acide acrylique d'origine renouvelable, en particulier la production d'acide acrylique à partir de glycérol, comportant une première étape de déshydratation du glycérol en acroléine suivie d'une étape d'oxydation en phase gaz de l'acroléine ainsi obtenue ; ou dans la production d'acide acrylique par déshydratation des acides 2-hydroxypropionique (acide lactique) ou 3-hydroxypropionique et de leurs esters ou les sels d'ammonium correspondants.

L'invention permet notamment de mettre en oeuvre un procédé de déshydratation du glycérol en acroléine. Dans ce type de procédé, le glycérol est alimenté sous forme d'une solution aqueuse avec une concentration de 20 à 95 % en masse. Plus le glycérol est concentré et plus le catalyseur a tendance à former du coke, nécessitant des régénérations fréquentes. Selon le procédé décrit dans la demande WO 2006/087083, la réaction est avantageusement mise en oeuvre en présence d'oxygène, l'ajout d'oxygène dans la réaction de déshydratation de glycérol permettant de prolonger la durée de vie du catalyseur et d'espacer les régénérations.

La réaction est typiquement effectuée à une température de 220 à 350°C et de préférence de 280 à 320°C ; et à une pression variant de la pression atmosphérique à quelques bars (par exemple 5).

Les catalyseurs utilisables pour cette réaction sont des catalyseurs acides, ayant notamment une acidité de Hammett inférieure à +2, tels que décrits par exemple dans les documents EP 1 848 681, WO 2009/12855, WO 2009/044081 ou WO 2010/046227. De nombreux catalyseurs acides peuvent convenir pour cette réaction. On citera les zircones phosphatées, les zircones tungstées, les zircones silicées, les oxydes de titane ou d'étain imprégnés de tungstate ou phosphotungstate ou silicotungstate, les alumines ou silices phosphatées, les hétéropolyacides ou sels d'hétéropolyacides, les phosphates de fer et les phosphates de fer comprenant un promoteur, les catalyseurs oxydes de titane imprégnés de tungstate ou phosphotungstate ou silicotungstate et/ou des sels alcalins de ces composés.

L'invention permet également de mettre en oeuvre un procédé de déshydratation de l'acide lactique ou de l'acide 3-hydroxypropionique (et de leurs esters correspondants) ou de leurs sels d'ammonium correspondants, en acide acrylique.

L'acide lactique a un point d'ébullition voisin de 217°C et l'acide 3-hydroxypropionique a un point d'ébullition de 279°C (valeur calculée). Les limites d'inflammabilité pour l'acide lactique dans l'air sont de 3,1 % (limite inférieure) et de 18 % (limite supérieure). L'ester méthylique de l'acide lactique a un point d'ébullition de 145°C, pour les limites d'inflammabilité de 1,1 et 3,6 % (ce qui donne une plus grande flexibilité d'utilisation que pour l'acide). L'ester méthylique de l'acide 3-hydroxypropionique a un point d'ébullition de 179°C (180°C en valeur calculée), l'ester éthylique de l'acide lactique a un point d'ébullition de 154°C, et des limites d'inflammabilité de 1,6 et 10,6 %. L'ester éthylique de l'acide 3-hydroxypropionique a un point d'ébullition de 187,5°C.

Pour ces réactions, on utilise une configuration de réacteur sensiblement identique à celle de la déshydratation du glycérol. Les conditions de déshydratation sont une température de 220 à 400°C, et de préférence de 250 à 350°C, et une pression de 0,5 à 5 bar.

Les catalyseurs qui peuvent convenir à ces réactions sont des catalyseurs acides, ayant notamment une acidité de Hammett inférieure à +2. Les catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides contenant notamment au moins un élément choisi parmi le groupe comprenant W, Mo et V. Parmi les oxydes mixtes, on peut citer particulièrement ceux à base de fer et de phosphore et ceux à base de césium, phosphore et tungstène.

D'autres catalyseurs qui peuvent aussi convenir pour ces réactions sont obtenus à partir de phosphates et/ou de sulfates des métaux alcalins, alcalino-terreux et des terres rares, et leurs mélanges. Dans ce groupe on trouve ainsi les phosphates et oxyphosphates de lanthane, les phosphates de sodium, les phosphates de calcium, le sulfate de calcium, le sulfate de magnésium, et les hydrogénophosphates correspondants. Le phosphate d'aluminium, le phosphate de bore. Toutes les matières actives citées ci-dessus peuvent être imprégnées ou enrobées sur tout type de support tel que : alumine, oxyde de titane, oxyde de zirconium, silice, mais aussi les oxydes mixtes correspondants, et le carbure de silicium.

La pression partielle en acide lactique ou 3-hydroxypropionique est généralement de 1 à 10 % et de préférence de 2 à 6 %.

L'invention permet également de mettre en oeuvre un procédé de déshydratation de l'acide 2-hydroxyisobutyrique ou de l'acide 3-hydroxyisobutyrique ou de leurs sels d'ammonium correspondants en acide méthacrylique.

Pour ce type de réaction, on utilise une configuration de réacteur sensiblement identique à celle de la déshydratation du glycérol. Les conditions de déshydratation sont une température de 200 à 400°C, et de préférence de 250 à 350°C, et une pression de 0,5 à 5 bar. Les catalyseurs qui peuvent convenir à cette réaction sont des catalyseurs acides, ayant notamment une acidité de Hammett inférieure à +2. Les catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides contenant notamment au moins un élément choisi parmi le groupe comprenant W, Mo et V. Parmi les oxydes mixtes, on peut citer particulièrement ceux à base de fer et de phosphore et ceux à base de césium, phosphore et tungstène.

Des catalyseurs qui peuvent aussi convenir pour cette réaction sont obtenus à partir de phosphates et/ou de sulfates des métaux alcalins, alcalino-terreux et des terres rares, et leurs mélanges. Dans ce groupe on trouve ainsi les phosphates et oxyphosphates de lanthane, les phosphates de sodium, les phosphates de calcium, le sulfate de calcium, le sulfate de magnésium, et les hydrogénophosphates correspondants. Le phosphate d'aluminium, le phosphate de bore. Toutes les matières actives citées ci-dessus peuvent être imprégnées ou enrobées sur tout type de support tel que : alumine, oxyde de titane, oxyde de zirconium, silice, mais aussi les oxydes mixtes correspondants, et le carbure de silicium.

La pression partielle en acide (2- ou 3-)hydroxyisobutyrique est généralement de 1 à 10 % et de préférence de 2 à 6 %.

Dans le cadre des réactions ci-dessus, le catalyseur subit une désactivation notamment par cokage. Cette désactivation peut toutefois être retardée par l'adjonction d'oxygène dans le flux réactionnel.

Par ailleurs, en phase de régénération, la régénération du catalyseur est effectuée en introduisant un flux de régénération à base d'oxygène ou d'air dans l'enceinte réactionnelle 1.

Les conditions de la régénération sont adaptées en fonction du type de la nature de la désactivation du catalyseur. Par exemple, dans le cas d'une désactivation par cokage, il existe différentes natures de coke et notamment du coke dit mou et du coke dit dur.

Le coke mou est riche en hydrogène et / ou s'élimine à relativement basse température, tandis que le coke dur est un coke qui a évolué (maturé) et est moins riche en hydrogène et nécessite des températures plus élevées pour amorcer sa combustion. La régénération nécessite en général d'atteindre une température suffisante dite température d'allumage. La température de régénération est de préférence supérieure à 300 °C, plus particulièrement supérieure à 325 °C, et de manière encore plus préférée supérieure à 350 °C. La température peut être modulée au cours de la régénération pour maintenir une température suffisante pour entretenir la régénération.

La pression est de préférence proche de la pression atmosphérique. C'est essentiellement la pression partielle en oxygène qui détermine la cinétique de combustion. La pression partielle en oxygène est de préférence de 0,01 à 1 bar, et plus particulièrement de 0,05 à 0,5 bar. La pression partielle en oxygène peut être modulée au cours de la régénération, par exemple en ajoutant de la vapeur d'eau, ou encore en ajoutant de l'azote, ou plus simplement en recyclant des gaz issus de la régénération, plus riches en $CO_2$. La composition du flux de régénération comprend de préférence, entre autres, de l'oxygène, de l'azote, de la vapeur d'eau, du $CO_2$, du monoxyde de carbone. L'air peut être utilisé avantageusement comme source d'oxygène, mais on peut aussi utiliser d'autres sources d'oxygène comme de l'air enrichi ou de l'air appauvri.

L'invention permet également de mettre en oeuvre des oxydations sélectives telles que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique, l'oxydation du benzène, du butène, du butanol ou du butane en anhydride maléique.

Dans le cas des réactions d'oxydation du méthanol en formaldéhyde ou diméthoxyméthane, et d'oxydation de l'éthanol en acétaldéhyde ou diéthoxydéthane, les catalyseurs qui peuvent convenir sont des oxydes mixtes, tels que les oxydes de molybdène et de fer, les oxydes de molybdène contenant des métaux choisis parmi le bismuth, le vanadium, le tungstène, le cuivre, le nickel, le cobalt. Les conditions opératoires sont une température comprise entre 200 et 350°C, de préférence entre 250 et 300°C ; une pression comprise entre 1 et 5 bar. La pression partielle en alcool peut varier dans une large gamme de 3 à 50 %, et de préférence de 5 à 40 %, suivant le type de produit recherché. Dans le cas où les aldéhydes sont les produits recherchés, la pression partielle en alcool est comprise entre 3 et 10 % et de préférence entre 5 et 9 %. Dans le cas où les acétals sont les produits recherchés, la pression partielle en alcool est comprise entre 10 et 50 % et de préférence entre 20 et 40 %.

Dans le cas des réactions d'oxydation de l'orthoxylène et du naphtalène en anhydride phtalique, les catalyseurs sélectionnés contiennent de préférence du vanadium, et de préférence de l'oxyde de vanadium supporté. Les conditions opératoires sont une pression de 1 à 5 bar, et des températures de réaction de 280 à 450°C.

Dans le cas des réactions d'oxydation du butane, des butènes, du butanol et du benzène en anhydride maléique, les catalyseurs qui conviennent contiennent du vanadium, sous forme d'oxyde de vanadium supporté ou sous forme de d'oxyde mixte vanadium-phosphore supporté. Les températures de réactions sont de 350 à 500°C, et les pressions de 1 à 5 bar.

Dans le cas des réactions d'oxydation du propylène en acroléine, de l'isobutène ou du tertiobutanol en méthacroléine, les catalyseurs qui conviennent sont constitués majoritairement de molybdène, et contiennent des éléments choisis parmi (mais pas exclusivement) les éléments suivants : nickel, fer, cobalt, tungstène, potassium, bismuth, antimoine, chrome. Les températures de réaction sont comprises entre 320 et 450°C. Les pressions totales sont comprises entre 1 et 5 bar. Les pressions partielles en composé hydrocarboné sont comprises entre 5 et 15 %, et le rapport $O_2$ / composé hydrocarboné en entrée de réacteur est compris entre 0,5 et 4, et de préférence entre 0,8 et 2, et encore plus de préférence entre 1 et 1,8, et encore plus préféré entre 1,2 et 1,6.

Dans le cas des réactions d'oxydation de l'acroléine en acide acrylique, et de la méthacroléine en acide méthacrylique, les catalyseurs qui conviennent sont constitués majoritairement de molybdène, et contiennent des éléments choisis parmi les éléments suivants (mais pas exclusivement) : vanadium, tungstène, cuivre, antimoine, niobium, strontium, phosphore, fer. Les températures de fonctionnement sont comprises entre 250 et 350°C, pour une pression totale de 1 à 5 bar. La pression partielle en aldéhyde est comprise entre 5 et 15 %, et le ratio $O_2$ / aldéhyde en entrée de réacteur est compris entre 0,3 et 1,2, et de préférence compris entre 0,5 et 1.

D'autres réactions d'oxydation pouvant être mises en oeuvre selon l'invention sont :

- La production d'acide acrylique à partir de propylène et d'oxygène, les coproduits étant l'acroléine, l'acide acétique, l'acide maléique, l'acide propionique, l'acétaldéhyde et l'acétone, par exemple à une température de 300 à 400°C et à une pression de 1 à 3 bar.
- La production d'oxyde d'éthylène à partir d'éthylène et d'oxygène, les coproduits étant l'acétaldéhyde et le formaldéhyde, par exemple à une température de 230 à 290°C et à une pression de 10 à 30 bar.
- La production de 1,2-dichloroéthane à partir d'éthylène, d'acide chlorhydrique et d'oxygène, les coproduits étant le monoxyde de carbone, le chloral et divers composés chlorés, par exemple à une température de 220 à 300°C et à une pression de 2 à 6 bar.
- La production d'acide téréphtalique à partir de p-xylène et d'oxygène, les coproduits étant l'anhydride maléique, l'acide o-toluique et l'acide benzoïque, par exemple à une température de 175 à 230°C et à une pression de 15 à 30 bar.

Le réacteur selon l'invention peut aussi convenir pour des réactions d'ammoxydation mettant en jeu des mélanges ammoniac-oxygène-inerte-composé hydrocarboné. Les composés hydrocarbonés qui peuvent être utilisés comprennent le propylène, l'isobutène, l'acroléine, la méthacroléine, mais aussi des composés aromatiques. Les réactions d'ammoxydation sont effectuées à une température de 50 à 100°C plus élevée que les températures d'oxydation correspondantes.

A titre d'exemple, on peut produire l'acrylonitrile (en coproduisant de l'acétonitrile, de l'acide hydrocyanique et du monoxyde de carbone) à partir de propylène et / ou propane, d'oxygène et d'ammoniac, par exemple à une température de 400 à 500°C et à une pression de 1 à 4 bar.

Pour l'ensemble des réactions d'oxydation ci-dessus, peu ou pas de coke est produit, et les durées de cycle se comptent généralement en mois, voire en années. L'intérêt de l'invention est dans ce cas principalement de compenser la migration des espèces catalytique comme le molybdène, et également de diminuer la perte de charge en restructurant le lit de catalyseur.

L'invention permet également de mettre en oeuvre un procédé de fluoration, c'est-à-dire un procédé de préparation d'un composé fluoré à partir d'un composé chloré. De préférence, la réaction est effectuée en faisant réagir le composé chloré avec du fluorure d'hydrogène (HF).

Le composé chloré peut être toute molécule possédant au moins un atome de chlore et le composé fluoré peut être toute molécule possédant au moins un atome de fluor.

De préférence, le composé chloré est un alcane ou un alcène en C2, ou en C3, ou en C4, ou en C5, linéaire ou ramifié (de préférence linéaire), comportant un ou plusieurs substituants choisis parmi F, Cl, I et Br (de préférence parmi F et Cl), au moins un des substituants étant Cl.

De préférence, le composé fluoré est un alcane ou un alcène en C2, ou en C3, ou en C4, ou en C5, linéaire ou ramifié (de préférence linéaire), comportant un ou plusieurs substituants choisis parmi F, Cl, I et Br (de préférence parmi F et Cl), au moins un des substituants étant F.

De manière plus particulièrement préférée, le composé chloré est un alcane ou un alcène en C3 comportant un ou plusieurs substituants choisis parmi F, Cl, I et Br (de préférence parmi F et Cl), au moins un des substituants étant Cl ; et le composé fluoré est un alcène en C3 comportant un ou plusieurs substituants choisis parmi F, Cl, I et Br (de préférence parmi F et Cl), au moins un des substituants étant F.

Alternativement, le composé chloré peut être un alcane ou un alcène en C4 comportant un ou plusieurs substituants choisis parmi F, Cl, I et Br (de préférence parmi F et Cl), au moins un des substituants étant Cl ; et le composé fluoré est un alcène en C4 comportant un ou plusieurs substituants choisis parmi F, Cl, I et Br (de préférence parmi F et Cl), au moins un des substituants étant F.

Selon un mode de réalisation, le composé fluoré est une hydrofluorooléfine (et ne comporte donc pas de substituant Cl).

De préférence, au cours de la réaction, au moins un substituant Cl du composé chloré est remplacé par un substituant F.

La conversion du composé chloré en le composé fluoré comprend la conversion directe (en une seule étape ou selon un seul ensemble de conditions opératoires) et la conversion indirecte (en deux ou plus de deux étapes ou en utilisant plus d'un ensemble de conditions opératoires).

Les réactions de fluoration plus particulièrement préférées sont les réactions :

- du 2-chloro-3,3,3-trifluoro-1-propène (HFCO-1233xf) en 2,3,3,3-tétrafluoro-1-propène (HFO-1234yf) ;

- du 1,1,1,2,3-pentachloropropane (HCC-240db) en 2,3,3,3-tétrafluoro-1-propène (HFO-1234yf) ;
- du 1,1,2,2,3-pentachloropropane (HCC-240aa) en 2,3,3,3-tétrafluoro-1-propène (HFO-1234yf) ;
- du 2,3-dichloro-1,1,1-trifluoropropane (HCFC-243db) en 2,3,3,3-tétrafluoro-1-propène (HFO-1234yf) ;
- du 1,1,2,3-tétrachloro-1-propène (HCO-1230xa) en 2,3,3,3-tétrafluoro-1-propène (HFO-1234yf) ;
- du 2,3,3,3-tétrachloro-1-propène (HCO-1230xf) en 2,3,3,3-tétrafluoro-1-propène (HFO-1234yf) ;
- du 1,1,1,2,3-pentachloropropane (HCC-240db) en 2-chloro-3,3,3-trifluoro-1-propène (HFCO-1233xf) ;
- du 1,1,2,2,3-pentachloropropane (HCC-240aa) en 2-chloro-3,3,3-trifluoro-1-propène (HFCO-1233xf) ;
- du 2,3-dichloro-1,1,1-trifluoropropane (HCFC-243db) en 2-chloro-3,3,3-trifluoro-1-propène (HFCO-1233xf) ;
- du 1,1,2,3 tétrachloro-1-propène (HCO-1230xa) en 2-chloro-3,3,3-trifluoro-1-propène (HFCO-1233xf) ;
- du 2,3,3,3 tétrachloro-1-propène (HCO-1230xf) en 2-chloro-3,3,3-trifluoro-1-propène (HFCO-1233xf).

La réaction de fluoration peut être effectuée avec un rapport molaire de HF typiquement de 3:1 à 150:1, avec un temps de contact de 6 à 100 s et à une pression de la pression atmosphérique à 20 bar. La température de la réaction peut être de 200 à 450°C.

Un exemple particulier de procédé permettant de préparer du HFO-1234yf à partir de HFCO-1233xf figure dans le document WO 2010/123154. Ce procédé peut être mis en oeuvre avec le réacteur selon l'invention.

Par ailleurs, la réaction peut être une réaction de déshydrohalogénation, comprenant la formation d'un composé insaturé (de préférence halogéné, et plus particulièrement fluoré) à partir d'un composé saturé halogéné (notamment chloré et / ou fluoré), et impliquant l'élimination d'une molécule du type HCl ou HF.

A titre d'exemples de déshydrohalogénation on peut citer celles conduisant au 2,3,3,3-tétrafluoro-1-propène (HFO-1234yf), et notamment les réactions :

- du 2-chloro-1,1,1,2-tétrafluoropropane (HCFC-244bb) en 2,3,3,3-tétrafluoro-1-propène (HFO-1234yf) ;
- du 1,1,1,2,2-pentafluoropropane (HFC-245cb) en 2,3,3,3-tétrafluoro-1-propène (HFO-1234yf) ;
- du 1,1,1,2,3-pentafluoropropane (HFC-245eb) en 2,3,3,3-tétrafluoro-1-propène (HFO-1234yf).

Le catalyseur utilisé pour les réactions ci-dessus mettant en jeu des composés fluorés peut être supporté ou non.

Il peut s'agir par exemple d'un catalyseur métallique, c'est à dire du type métal élémentaire, oxyde métallique, halogénure métallique et / ou sel métallique), en particulier un oxyde de métal de transition ou un halogénure ou un oxyhalogénure d'un tel métal.

Il peut s'agir notamment d'halogénure d'antimoine, d'halogénure d'étain, d'halogénure de thallium, d'halogénure de thallium, d'halogénure de fer et de combinaison de ceux-ci. Les chlorures et fluorures métalliques sont préférés, par exemple $SbCl_5$, $SbCl_3$, $SbF_5$, $SnCl_4$, $TiCl_4$, $FeCl_3$ et les combinaisons de ceux-ci.

D'autres catalyseurs appropriés sont ceux à base de chrome, tels que l'oxyfluorure de chrome, les oxydes de chrome tels que $Cr_2O_3$ (éventuellement soumis à des traitements de fluoration), les fluorures de chrome et les combinaisons de ceux-ci.

D'autres catalyseurs possibles sont ceux à base d'aluminium (par exemple $AlF_3$, $Al_2O_3$ et oxyfluorure d'aluminium).

Le catalyseur peut être choisi parmi l'alumine fluorée, le dioxyde de titane fluoré, l'acier inoxydable fluoré, le charbon actif et le graphite.

Il peut s'agir d'un mélange de chrome et de magnésium (sous forme élémentaire, saline, oxyde, ou halogénure) ; ou d'un mélange de chrome et d'un autre métal (sous forme élémentaire, saline, oxyde, ou halogénure).

Il peut également s'agir d'un catalyseur comprenant un métal sur un support.

Le métal peut être choisi parmi les groupes 3, 4, 5, 6, 7, 8, 9 et 13 du tableau périodique, et peut notamment être Al, Cr, Mn, Co, Ni, Zn, Ti, V, Ru, Rh, Pd, Os, Ir, Pt, Zr, Mo, Re, Sc, Y, La, Hf, Cu, Ag, Au, Ge, Sn, Pb ou Mg, notamment Al, Cr, Mn, Ni ou Co. Il peut s'agir d'un lanthanide (métaux 58 à 71 du tableau périodique). Le métal du catalyseur peut être converti en dérivés métalliques lors de son activation ou de sa régénération, par exemple en oxyde, halogénure (fluorure, bromure, chlorure), oxyhalogénure ou pseudohalogénure (cyanure, cyanate, thiocyanate).

Le support peut être choisi parmi l'aluminium, les halogénures d'aluminium et les oxyhalogénures d'aluminium, l'alumine, l'alumine activée, l'alumine fluorée, le fluorure d'aluminium, le charbon actif (fluoré ou non) et le graphite (éventuellement fluoré).

Le catalyseur peut être préparé par exemple par immersion du support dans une solution de composé soluble (par exemple nitrate ou chlorure) du métal, ou alternativement la solution peut être pulvérisée sur le support. Le support peut être séché et mis en contact avec un agent d'halogénation sous forme vapeur (par exemple fluorure d'hydrogène, acide chlorhydrique, chlorofluorohydrocarbure, ou encore $SiF_4$, $CCl_3F$, $CCl_2F$, $CHF_3$, $CCl_2FCClF_2$) avec chauffage afin d'halogéner partiellement ou complètement le support ou le métal.

Des exemples de catalyseurs supportés sont $FeCl_3$ supporté sur du carbone, l'alumine supportée sur du carbone, le fluorure d'aluminium supporté sur du carbone, l'alumine fluorée supportée sur du carbone, le fluorure de magnésium supporté sur du fluorure d'aluminium, plus généralement les métaux (métaux élémentaires, oxydes métalliques, halogénures métalliques et / ou sels métalliques) supportés sur du fluorure d'aluminium, les métaux supportés sur de l'alumine, les métaux supportés sur du carbone, les mélanges de métaux.

D'autres exemples de catalyseurs supportés sont : un halogénure de magnésium ou un halogénure de zinc supporté sur $Cr_2O_3$, un halogénure de chrome III supporté sur du carbone, un mélange de chrome et magnésium (sous forme élémentaire, oxyde, halogénure ou saline) supporté sur du graphite, un mélange de chrome et d'un autre métal (sous forme élémentaire, saline, oxyde, ou halogénure) supporté sur du graphite ou de l'alumine ou un halogénure d'aluminium tel que le fluorure d'aluminium.

La teneur métallique totale du catalyseur supporté est de préférence de 0,1 à 20 % en masse, par exemple de 0,1 à 10 % (par rapport à la masse totale du catalyseur).

Un mode de réalisation préféré utilise un catalyseur particulier, qui est un catalyseur mixte supporté, contenant à la fois du chrome et du nickel. Le rapport molaire Cr:Ni (par rapport aux éléments métalliques) est généralement de 0,5 à 5, par exemple de 0,7 à 2, notamment voisin de 1. Le catalyseur peut contenir de 0,5 à 20 % de chrome et de 0,5 à 20 % de nickel en masse, de préférence de 2 à 10 % de chaque métal. On peut faire référence à cet égard au document WO 2009/118628, et en particulier à la description du catalyseur de la p.4, l.30 à la p.7, l.16.

Un catalyseur intéressant est un catalyseur à base de chrome comprenant un composé chromé du type oxyde de chrome cristallin sous forme alpha, dans lequel de 0,05 à 6 % des atomes environ du réseau d'oxyde de chrome alpha sont remplacés par des atomes de cobalt trivalents (éventuellement traité par un agent de fluoration). On renvoie au document US 2005/0228202 à ce sujet.

Le catalyseur peut subir une fluoration. A titre d'exemple de fluoration, on peut préparer une alumine fluorée en mettant en contact une alumine avec du fluorure d'hydrogène avec chauffage, ou en pulvérisant une solution aqueuse de fluorure d'hydrogène à température ambiante ou en immergeant une alumine en solution, puis en séchant. La fluoration du catalyseur peut être mise en oeuvre dans le réacteur selon l'invention ou non. La température lors de la fluoration est généralement de 200 à 450°C.

Le catalyseur peut éventuellement contenir une faible teneur d'un ou plusieurs co-catalyseurs tels que des sels de Co, Zn, Mn, Mg, V, Mo, Te, Nb, Sb, Ta, P et Ni. Un co-catalyseur préféré est le nickel. Un autre co-catalyseur préféré est le magnésium.

Par exemple, un catalyseur à base de chrome non supporté peut éventuellement contenir une faible teneur d'un ou plusieurs co-catalyseurs choisis parmi le cobalt, le nickel, le zinc ou le manganèse, et être préparé par des procédés connus en soi, tels que l'imprégnation, le mélange de poudres et autres.

La quantité de co-catalyseur, lorsqu'il est présent, peut varier de 1 à 10 % en masse, de préférence de 1 à 5 % en masse. Le co-catalyseur peut être ajouté au catalyseur par des procédés connus en soi tels que l'adsorption à partir d'une solution aqueuse ou organique, suivie de l'évaporation du solvant. Un catalyseur préféré est de l'oxyde de chrome pur avec du nickel ou du zinc en tant que co-catalyseur. Alternativement, le co-catalyseur peut être physiquement mélangé avec le catalyseur par broyage pour produire un mélange fin.

Un autre catalyseur préféré est un catalyseur mixte chrome / nickel supporté sur de l'alumine fluorée. Le document US 5,731,481 décrit un procédé de préparation de cet autre catalyseur.

Avant activation, le catalyseur est soumis à une étape de séchage, de préférence avec un gaz de séchage tel que l'azote. L'étape de séchage peut être effectuée à une pression allant de la pression atmosphérique à 20 bar. La température du catalyseur lors de l'étape de séchage peut aller de 20°C à 400°C, de préférence de 100 à 200°C.

L'activation du catalyseur peut être de préférence effectuée avec HF ou un fluoro ou hydrofluoroalcane et / ou un agent oxydant (oxygène ou chlore de préférence). La régénération du catalyseur peut être effectuée avec un agent oxydant (oxygène ou chlore de préférence) et éventuellement HF, en une ou plusieurs étapes.

Afin d'allonger la durée de fonctionnement du catalyseur, il est possible d'ajouter un agent oxydant (oxygène ou chlore de préférence) lors de la phase de production, par exemple à raison de 0,05 à 15 mol% par rapport au mélange d'agent oxydant et de composé chloré.

La température lors de l'étape de régénération peut être de 250 à 500°C environ et la pression de la pression atmosphérique à environ 20 bar. Lorsqu'on utilise HF en combinaison avec de l'oxygène, la proportion d'oxygène peut varier de 2 à 98 mol% par rapport au mélange HF / oxygène.

De manière générale, et quelle que soit la réaction envisagée, il est préféré que la différence de température moyenne entre la phase de réaction et la phase de régénération soit inférieure à 150°C, ou inférieure à 100°C, voire inférieure ou égale à 75°C ou 50°C. De la sorte, on améliore la productivité, en atteignant plus rapidement les conditions d'équilibre et en limitant les réactions parasites en début de phase de réaction.

Il est possible de prévoir une phase de purge intermédiaire entre la phase de réaction et la phase de régénération, et/ou entre la phase de régénération et la phase de réaction, par exemple par balayage de l'enceinte réactionnelle 1 à l'azote ou à la vapeur d'eau (soit dans le sens du flux réactionnel, soit dans le sens du flux de régénération). Une telle phase de purge peut toutefois être inutile si de l'oxygène est de toute façon présent dans le flux réactionnel. L'utilisation d'une purge par de la vapeur d'eau avant la phase de réaction peut permettre de préchauffer le réacteur, du fait de l'adsorption d'eau exothermique sur le catalyseur (de l'eau étant de toute façon produite dans le cadre des réactions de déshydratation). L'adsorption de vapeur d'eau sur un catalyseur anhydre libère une quantité importante de chaleur (notamment dans le cas des zéolithes), ce qui contribue à élever de manière importante la température du solide. Après cette phase initiale, la température du solide revient à la normale, et il est souhaitable d'attendre ce retour avant d'injecter les réactifs pour éviter tout emballement de la réaction.

Dans un mode préféré, à la fin d'un cycle de réaction on procède à une purge du réacteur soit dans le sens du flux de la réaction soit dans le sens opposé avec un gaz inerte tel que de l'azote, du $CO_2$, des gaz de combustion ou de la vapeur d'eau. Au cours de cette phase de purge, d'une durée relativement courte, la température du lit catalytique est maintenue à la même température, c'est-à-dire dans une gamme de plus ou moins 10°C autour de la température atteinte à la fin du cycle de réaction.

A l'issue de la phase de purge, le flux de gaz de régénération est introduit à partir de l'extrémité opposée à l'entrée des gaz de réaction. Le flux de régénération contient de l'oxygène, qui est dilué dans un gaz inerte contenant de l'azote, du $CO_2$ ou de la vapeur d'eau. La pression partielle en oxygène au cours de la phase de régénération peut être modulée et notamment peut démarrer avec une faible teneur en oxygène, inférieure à 20 %, et de préférence inférieure à 10 %, qui est ensuite progressivement augmentée pour maintenir la régénération du catalyseur au travers du lit de catalyseur. Dans ce cas, la régénération du catalyseur peut être pilotée par la température du point le plus chaud dans le réacteur qui est maintenue à moins de 500°C, et de préférence moins de 450°C, et de manière encore plus préférée inférieure à 400°C. La régénération peut encore être pilotée en suivant la concentration en oxygène résiduel sortant du réacteur et en s'assurant que la conversion de l'oxygène reste inférieure à 95 %, et de préférence inférieure à 90 %.

A l'issue de la phase de régénération, on opère une purge rapide du réacteur, en s'assurant que la température du lit catalytique reste dans une gamme compatible avec le démarrage d'une phase de réaction après la fin de la combustion du coke. La purge peut être effectuée avec un gaz contenant par exemple de l'azote, du $CO_2$, des gaz de combustion ou de la vapeur d'eau. De préférence la purge est effectuée dans le sens des gaz de réaction.

Dans le cas de réactions impliquant de l'HF, les purges sont de préférence faites en absence de vapeur d'eau pour limiter les risques de corrosion.

Les avantages de l'invention sont maintenant illustrés de façon non limitative pour un procédé de déshydratation de glycérol en acroléine.

## EXEMPLES

Exemple 1 (selon l'invention)

Un tube réactionnel (1) en inox 316L, de diamètre intérieur 22 mm et de hauteur 1 m est immergé verticalement dans un bain de sel (mélange eutectique $KNO_3$, $NaNO_3$, $NaNO_2$) pourvu d'une résistance chauffante et agité par bullage d'azote. Ce dispositif permet d'assurer efficacement les échanges thermiques avec le réacteur. La température à l'intérieur du réacteur est suivie par 14 thermocouples situés dans une canne thermométrique de diamètre extérieur 4 mm positionnée au centre du tube réactionnel. Le tube réactionnel est chargé avec 0,337 litres d'un catalyseur $PW/TiO_2$, préparé selon l'exemple 1 de la demande de brevet WO 2013/18915.

Dans une phase de réaction, une pompe volumétrique et 2 régulateurs de débit massique permettent d'envoyer respectivement un mélange glycérol/eau, de l'azote et de l'air via une ligne (5) dans un vaporiseur chauffé électriquement connecté à l'extrémité (2) du réacteur située au sommet de celui-ci. L'extrémité (3) du réacteur située au bas de celui-ci est connectée à une vanne automatique asservie à un capteur de pression qui permet de maintenir la pression désirée dans le réacteur. Le flux gazeux sortant de cette vanne peut être envoyé à des fins analytiques vers une colonne d'abattage à l'eau. L'effluent liquide ainsi collecté en pied de la colonne sur une durée d'une dizaine de minutes peut être pesé et analysé par chromatographie en phase gaz.

Pour réaliser une phase de régénération à flux inversé, on a installé, entre la vanne automatique et l'extrémité basse (3) du réacteur, une ligne (8) alimentée par 2 régulateurs de débit massique d'azote et d'air. Une ligne (7) permettant d'évacuer un flux gazeux est connectée sur le vaporiseur au niveau de l'arrivée de la ligne (5).

Dans les phases de réaction, des vannes permettent de fermer les lignes (8) et (7).

Dans les phases de régénération à flux inversé, la ligne (5) est fermée par une vanne et la vanne automatique connectée à l'extrémité (3) est en position fermée.

De l'instant t=0 et jusqu'à t=24h, on réalise une phase de réaction en envoyant un flux continu de 675 Nl/h (normaux litres par heure) d'un mélange glycérol / eau / $N_2$ / $O_2$ de ratios volumiques 6 / 29 / 62 / 3 à 295°C à l'entrée haute (2) du réacteur. Le bain de sel fondu est maintenu à 295°C et la pression du flux gazeux en sortie du réacteur est maintenue à 2,3 bars absolus via la vanne automatique.

De t=24h à t=24h05, la pression dans le réacteur est rabaissée à la pression atmosphérique et le réacteur est balayé par un flux de 416 Nl/h d'azote via la ligne (5). On débute alors la phase de régénération en flux inversé en fermant la vanne automatique connectée à l'extrémité (3) du réacteur et la ligne (5) et en injectant à pression atmosphérique à l'extrémité basse (3) du réacteur via la ligne (8) un flux de 169 Nl/h d'un mélange air / azote à 3,3% volumique d'oxygène. La température du sel fondu est portée à 315°C. On observe que la température dans le lit catalytique passe par un maximum de 328°C en début de régénération (« point chaud ») puis décroît. A partir de t=34h, on injecte 169 Nl/h d'un mélange air/azote à 7% volumique d'oxygène. A t=41h30, la température du sel fondu est rabaissée à 295°C.

A t=42h, on repasse en configuration de réaction en fermant les lignes (7) et (8). Un flux continu de 675 Nl/h (normaux litres par heure) d'un mélange glycérol / eau / $N_2$ / $O_2$ de ratios volumiques 6 / 29 / 62 / 3 à 295°C est envoyé à l'entrée haute (2) du réacteur. Le bain de sel fondu du réacteur (1) est maintenu à 295°C et la pression du flux gazeux en sortie du réacteur est maintenue à 2,3 bars absolus via la vanne automatique. Très rapidement après le démarrage de la réaction, la température mesurée dans le réacteur passe par un maximum de 359°C (point chaud) avant de décroître. La phase de réaction est poursuivie jusqu'à t=66h. Le flux gazeux sortant est envoyé à différents moments vers la colonne d'abattage à l'eau.

De la même façon que précédemment, on reproduit une phase de régénération à flux inversé de t=66h à t=84h et une phase de réaction de t=84h à t=108h. L'analyse de l'effluent liquide issu de la colonne d'absorption du flux gazeux sortant du réacteur a permis de déterminer le taux de conversion du glycérol et le rendement en acroléine, selon les formules suivantes :

Conversion du glycérol (%) = 100 – débit molaire de glycérol dans l'effluent gazeux / débit molaire de glycérol introduit dans le réacteur.

Rendement en acroléine (%) = débit molaire d'acroléine dans l'effluent gazeux / débit molaire de glycérol introduit dans le réacteur.

Les résultats sont rassemblés dans le tableau 1.

Tableau 1

| | Temps (h) | Température du réacteur (bain de sel) | Point chaud maximum | Conversion glycérol,% | Rendement acroléine,% |
|---|---|---|---|---|---|
| Régénération à flux inversé | 24 -42h | 315°C | 328°C | - | - |
| Réaction | 42h15 - 42h25 | 295°C | 359°C | > 99 | 72 |
| | 65 h30 - 65h40 | | | > 99 | 70 |
| Régénération à flux inversé | 66h - 84h | 315°C | 327°C | - | - |
| Réaction | 84h15 - 84h25 | 295°C | 356°C | > 99 | 72 |
| | 107h30 - 107h40 | | | > 99 | 70 |

Exemple 2 (comparatif)

On reproduit les conditions de l'exemple 1 entre 0 et 24h.

L'étape de régénération est effectuée de t=24h à t=42h sans inversion de flux.

De t=24h à t=24h05, la pression dans le réacteur est rabaissée à la pression atmosphérique et le réacteur est balayé par un flux de 416 Nl/h d'azote via la ligne (5). On débute alors la phase de régénération en injectant à pression atmosphérique à l'extrémité haute (2) du réacteur via la ligne (5) un flux de 169 Nl/h d'un mélange air / azote à 3,3% volumique d'oxygène. La température du sel fondu est portée à 315°C. On observe que la température dans le lit catalytique passe par un maximum de 337°C en début de régénération (« point chaud ») puis décroît. A partir de t=34h, on injecte 169 Nl/h d'un mélange air/azote à 7% volumique d'oxygène. A t=41h30, la température du sel fondu est rabaissée à 295°C.

On reproduit alors les conditions de l'exemple 1 entre 42h et 66h. Très rapidement après le démarrage de la réaction, la température mesurée dans le réacteur passe par un maximum de 378°C (point chaud) avant de décroître.

Les résultats sont rassemblés dans le tableau 2.

Tableau 2

| | Temps (h) | Température du réacteur (bain de sel) | Point chaud maximum | Conversion glycérol,% | Rendement acroléine,% |
|---|---|---|---|---|---|
| Régénération sans inversion de flux | 24h -42h | 315°C | 337°C | - | - |
| Réaction | 42h15 - 42h25 | 295°C | 378°C | > 99 | 61 |
| | 65h30 - 65h40 | | | > 99 | 70 |

On constate que la régénération à flux inversé permet de diminuer le point chaud maximum à la fois au démarrage de la réaction (328 et 327°C dans l'exemple 1 versus 337°C dans l'exemple 2 comparatif) et au démarrage de la régénération (356 et 359°C dans l'exemple 1 versus 378°C dans l'exemple 2 comparatif). De plus le rendement 15 minutes après le début de la phase de réaction est nettement amélioré (72% dans l'exemple 1 versus 61% dans l'exemple 2 comparatif).

## Revendications

**1.** Procédé de réaction chimique mis en oeuvre dans une enceinte réactionnelle (1) contenant un lit de catalyseur, l'enceinte réactionnelle comprenant une première extrémité (2) et une deuxième extrémité (3) opposée à la première extrémité (2), le procédé comprenant de manière alternée :

- une phase de réaction, comprenant le passage d'un flux réactionnel dans l'enceinte réactionnelle (1) de sa première extrémité (2) vers sa deuxième extrémité (3) ; et
- une phase de régénération du catalyseur, comprenant le passage d'un flux de régénération dans l'enceinte réactionnelle (1) de sa deuxième extrémité (3) vers sa première extrémité (2),

**caractérisé en ce que** l'enceinte réactionnelle (1) est constituée d'une pluralité de compartiments réactionnels distincts contenant un lit de catalyseur et comprenant un système d'échange thermique entre eux, et ledit procédé étant :

- un procédé de déshydratation du glycérol en acroléine ; ou
- un procédé de déshydratation de l'acide lactique ou du lactate d'ammonium en acide acrylique ; ou
- un procédé de déshydratation de l'acide 3-hydroxypropionique ou de son sel d'ammonium en acide acrylique ; ou
- un procédé de déshydratation de l'acide 2-hydroxyisobutyrique ou de l'acide 3-hydroxyisobutyrique ou de leurs sels d'ammonium correspondants en acide méthacrylique ; ou
- un procédé de conversion d'un composé chloré en un composé fluoré, de préférence un procédé de préparation d'une hydrofluorooléfine ou d'un hydrofluorocarbure, notamment un procédé de préparation d'un fluoropropène, et de manière tout particulièrement préférée un procédé de préparation du 2,3,3,3-tétrafluoropropène ; ou
- un procédé d'oxydation sélective telle que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique ; l'oxydation du benzène, du butène, du butanol ou du butane en anhydride maléique ; l'oxydation du propylène en acroléine ; l'oxydation de l'isobutène ou du tertiobutanol en méthacroléine.

**2.** Procédé selon la revendication 1, dans lequel le flux de régénération comprend de l'oxygène, et la phase de régénération du catalyseur comprend la combustion de coke déposé sur le catalyseur.

**3.** Procédé selon la revendications 1 ou 2, dans lequel la phase de réaction présente une durée supérieure ou égale à 15 minutes, de préférence supérieure ou égale à 1 heure ou supérieure ou égale à 15 heures ; et / ou la phase de régénération présente une durée supérieure ou égale à 15 minutes, de préférence supérieure ou égale à 5 heures.

4. Procédé selon l'une des revendications 1 à 3, dans lequel la phase de réaction et la phase de régénération ont des durées prédéterminées ; ou dans lequel la durée de la phase de réaction et / ou la durée de la phase de régénération dépendent de la mesure d'un paramètre tel qu'une température dans le réacteur ou la teneur d'un composé dans un flux sortant du réacteur.

5. Utilisation d'un réacteur chimique comprenant :

- une enceinte réactionnelle (1) contenant un lit de catalyseur, l'enceinte réactionnelle comprenant une première extrémité (2) et une deuxième extrémité (3) opposée à la première extrémité (2), ainsi qu'une pluralité de compartiments réactionnels distincts contenant un lit de catalyseur avec un système d'échange thermique entre eux ;
- une conduite d'amenée de flux réactionnel (5) connectée en entrée de l'enceinte réactionnelle (1) à sa première extrémité (2) ;
- une conduite de soutirage de flux réactionnel (6) connectée en sortie de l'enceinte réactionnelle (1) à sa deuxième extrémité (3) ;
- une conduite d'amenée de flux de régénération (8) connectée en entrée de l'enceinte réactionnelle (1) à sa deuxième extrémité (3) ;
- une conduite de soutirage de flux de régénération (7) connectée en sortie de l'enceinte réactionnelle (1) à sa première extrémité (2) ;
- des moyens de commutation adaptés pour permettre alternativement le passage d'un flux réactionnel dans l'enceinte réactionnelle (1) depuis la conduite d'amenée de flux réactionnel (5) jusqu'à la conduite de soutirage de flux réactionnel (6), et le passage d'un flux de régénération depuis la conduite d'amenée de flux de régénération (8) jusqu'à la conduite de soutirage de flux de régénération (7) ;

pour mettre en oeuvre un procédé de réaction chimique comprenant de manière alternée :

- une phase de réaction, comprenant le passage d'un flux réactionnel dans l'enceinte réactionnelle (1) de sa première extrémité (2) vers sa deuxième extrémité (3) ; et
- une phase de régénération du catalyseur, comprenant le passage d'un flux de régénération dans l'enceinte réactionnelle (1) de sa deuxième extrémité (3) vers sa première extrémité (2) ;

le réacteur étant de préférence un réacteur à plaques ou un réacteur multitubulaire,
et ledit procédé de réaction chimique étant :

- un procédé de déshydratation du glycérol en acroléine ; ou
- un procédé de déshydratation de l'acide lactique ou du lactate d'ammonium en acide acrylique ; ou
- un procédé de déshydratation de l'acide 3-hydroxypropionique ou de son sel d'ammonium en acide acrylique ; ou
- un procédé de déshydratation de l'acide 2-hydroxyisobutyrique ou de l'acide 3-hydroxyisobutyrique ou de leurs sels d'ammonium correspondants en acide méthacrylique ; ou
- un procédé de conversion d'un composé chloré en un composé fluoré, de préférence un procédé de préparation d'une hydrofluorooléfine ou d'un hydrofluorocarbure, notamment un procédé de préparation d'un fluoropropène, et de manière tout particulièrement préférée un procédé de préparation du 2,3,3,3-tétrafluoropropène ; ou
- un procédé d'oxydation sélective telle que l'oxydation du méthanol en formaldéhyde ou en diméthoxyméthane ; l'oxydation de l'éthanol en acétaldéhyde ou en diethoxyéthane ; l'oxydation de l'orthoxylène ou du naphtalène en anhydride phtalique ; l'oxydation du benzène, du butène, du butanol ou du butane en anhydride maléique ; l'oxydation du propylène en acroléine ; l'oxydation de l'isobutène ou du tertiobutanol en méthacroléine.

## Patentansprüche

1. Verfahren zur chemischen Umsetzung, das in einer Reaktionskammer (1), die eine Katalysatorschüttung enthält, durchgeführt wird, wobei die Reaktionskammer ein erstes Ende (2) und ein zweites Ende (3), das dem ersten Ende (2) gegenüberliegt, aufweist, wobei das Verfahren alternierend Folgendes umfasst:

- eine Reaktionsphase, umfassend das Durchleiten eines Reaktionsstroms durch die Reaktionskammer (1) von ihrem ersten Ende (2) zu ihrem zweiten Ende (3); und

- eine Katalysatorregenerationsphase, umfassend das Durchleiten eines Regenerationsstroms durch die Reaktionskammer (1) von ihrem zweiten Ende (3) zu ihrem ersten Ende (2),

**dadurch gekennzeichnet, dass** die Reaktionskammer (1) aus mehreren separaten Reaktionskompartimenten, die eine Katalysatorschüttung enthalten und ein System zum Wärmeaustausch dazwischen umfassen, besteht, und wobei es sich bei dem Verfahren um:

- ein Verfahren zur Dehydratisierung von Glycerin zu Acrolein; oder

- ein Verfahren zur Dehydratisierung von Milchsäure oder Ammoniumlactat zu Acrylsäure; oder

- ein Verfahren zur Dehydratisierung von 3-Hydroxypropionsäure oder deren Ammoniumsalz zu Acrylsäure; oder

- ein Verfahren zur Dehydratisierung von 2-Hydroxyisobuttersäure oder 3-Hydroxyisobuttersäure oder deren entsprechenden Ammoniumsalzen zu Methacrylsäure; oder

- ein Verfahren zur Umwandlung einer Chlorverbindung in eine Fluorverbindung, vorzugsweise ein Verfahren zur Herstellung eines Hydrofluorolefins oder eines teilfluorierten Fluorkohlenwasserstoffs, insbesondere ein Verfahren zur Herstellung eines Fluorpropens und ganz besonders bevorzugt ein Verfahren zur Herstellung von 2,3,3,3-Tetrafluorpropen; oder

- ein Verfahren zur selektiven Oxidation wie zur Oxidation von Methanol zu Formaldehyd oder zu Dimethoxymethan; zur Oxidation von Ethanol zu Acetaldehyd oder zu Diethoxyethan; zur Oxidation von ortho-Xylol oder Naphthalin zu Phthalsäureanhydrid; zur Oxidation von Benzol, Buten, Butanol oder Butan zu Maleinsäureanhydrid; zur Oxidation von Propylen zu Acrolein oder zur Oxidation von Isobuten oder tert-Butanol zu Methacrolein

handelt.

2. Verfahren nach Anspruch 1, bei dem der Regenerationsstrom Sauerstoff umfasst und die Katalysatorregenerationsphase die Verbrennung von auf dem Katalysator abgeschiedenem Koks umfasst.

3. Verfahren nach Anspruch 1 oder 2, bei dem die Reaktionsphase eine Dauer größer oder gleich 15 Minuten, vorzugsweise größer oder gleich 1 Stunde oder größer oder gleich 15 Stunden, aufweist und/oder die Regenerationsphase eine Dauer größer oder gleich 15 Minuten, vorzugsweise größer oder gleich 5 Stunden, aufweist.

4. Verfahren nach einem der Ansprüche 1 bis 3, bei dem die Reaktionsphase und die Regenerationsphase vorgegebene Dauern aufweisen; oder wobei die Dauer der Reaktionsphase und/oder der Dauer der Regenerationsphase von der Messung eines Parameters wie einer Temperatur im Reaktor oder dem Gehalt einer Verbindung in dem aus dem Reaktor austretenden Strom abhängt.

5. Verwendung eines chemischen Reaktors mit:

- einer Reaktionskammer (1), die eine Katalysatorschüttung enthält, wobei die Reaktionskammer ein erstes Ende (2) und ein zweites Ende (3), das dem ersten Ende (2) gegenüberliegt, sowie mehrere separate Reaktionskompartimente, die eine Katalysatorschüttung enthalten, mit einem System zum Wärmeaustausch dazwischen aufweist;

- eine Leitung zum Zuführen von Reaktionsstrom (5), die mit dem Eingang der Reaktionskammer (1) an ihrem ersten Ende (2) verbunden ist;

- eine Leitung zum Austragen von Reaktionsstrom (6), die mit dem Ausgang der Reaktionskammer (1) an ihrem zweiten Ende (3) verbunden ist;

- eine Leitung zum Zuführen von Regenerationsstrom (8), die mit dem Eingang der Reaktionskammer (1) an ihrem zweiten Ende (3) verbunden ist;

- eine Leitung zum Austragen von Regenerationsstrom (7), die mit dem Ausgang der Reaktionskammer (1) an ihrem ersten Ende (2) verbunden ist;

- Umschaltmittel, die dazu ausgelegt sind, das Durchleiten eines Reaktionsstroms durch die Reaktionskammer (1) von der Leitung zum Zuführen von Reaktionsstrom (5) zur Leitung zum Austragen von Reaktionsstrom (6) und das Durchleiten eines Regenerationsstroms von der Leitung zum Zuführen von Regenerationsstrom (8) bis zur Leitung zum Austragen von Regenerationsstrom (7) zu ermöglichen;

zur Durchführung eines Verfahrens zur chemischen Umsetzung, das alternierend Folgendes umfasst:

- eine Reaktionsphase, umfassend das Durchleiten eines Reaktionsstroms durch die Reaktionskammer (1) von ihrem ersten Ende (2) zu ihrem zweiten Ende (3); und
- eine Katalysatorregenerationsphase, umfassend das Durchleiten eines Regenerationsstroms durch die Reaktionskammer (1) von ihrem zweiten Ende (3) zu ihrem ersten Ende (2),

wobei es sich bei dem Reaktor vorzugsweise um einen Plattenreaktor oder einen Mehrrohrreaktor handelt und wobei es sich bei dem Verfahren zur chemischen Umsetzung um:

- ein Verfahren zur Dehydratisierung von Glycerin zu Acrolein; oder
- ein Verfahren zur Dehydratisierung von Milchsäure oder Ammoniumlactat zu Acrylsäure; oder
- ein Verfahren zur Dehydratisierung von 3-Hydroxypropionsäure oder deren Ammoniumsalz zu Acrylsäure; oder
- ein Verfahren zur Dehydratisierung von 2-Hydroxyisobuttersäure oder 3-Hydroxyisobuttersäure oder deren entsprechenden Ammoniumsalzen zu Methacrylsäure; oder
- ein Verfahren zur Umwandlung einer Chlorverbindung in eine Fluorverbindung, vorzugsweise ein Verfahren zur Herstellung eines Hydrofluorolefins oder eines teilfluorierten Fluorkohlenwasserstoffs, insbesondere ein Verfahren zur Herstellung eines Fluorpropens und ganz besonders bevorzugt ein Verfahren zur Herstellung von 2,3,3,3-Tetrafluorpropen; oder
- ein Verfahren zur selektiven Oxidation wie zur Oxidation von Methanol zu Formaldehyd oder zu Dimethoxymethan; zur Oxidation von Ethanol zu Acetaldehyd oder zu Diethoxyethan; zur Oxidation von ortho-Xylol oder Naphthalin zu Phthalsäureanhydrid; zur Oxidation von Benzol, Buten, Butanol oder Butan zu Maleinsäureanhydrid; zur Oxidation von Propylen zu Acrolein oder zur Oxidation von Isobuten oder tert-Butanol zu Methacrolein

handelt.

## Claims

1. Chemical reaction process performed in a reaction chamber (1) containing a catalyst bed, the reaction chamber comprising a first end (2) and a second end (3) opposite the first end (2), the process alternately comprising:

   - a reaction phase, comprising the passage of a reaction stream in the reaction chamber (1) from its first end (2) to its second end (3); and
   - a catalyst regeneration phase, comprising the passage of a regeneration stream in the reaction chamber (1) from its second end (3) to its first end (2),

   **characterized in that** the reaction chamber (1) consists of a plurality of separate reaction compartments containing a catalyst bed and comprising a system for heat exchange therebetween, and said process being:

   - a process for the dehydration of glycerol to give acrolein; or
   - a process for the dehydration of lactic acid or ammonium lactate to give acrylic acid; or
   - a process for the dehydration of 3-hydroxypropionic acid or the ammonium salt thereof to give acrylic acid; or
   - a process for the dehydration of 2-hydroxyisobutyric acid or 3-hydroxyisobutyric acid or the corresponding ammonium salts thereof to give methacrylic acid; or
   - a process for the conversion of a chlorinated compound into a fluorinated compound, preferably a process for the preparation of a hydrofluoroolefin or of a hydrofluorocarbon, in particular a process for the preparation of a fluoropropene and very particularly preferably a process for the preparation of 2,3,3,3-tetrafluoropropene; or
   - a selective oxidation process such as the oxidation of methanol to give formaldehyde or dimethoxymethane; the oxidation of ethanol to give acetaldehyde or diethoxyethane; the oxidation of ortho-xylene or naphthalene to give phthalic anhydride; the oxidation of benzene, butene, butanol or butane to give maleic anhydride; the oxidation of propylene to give acrolein; or the oxidation of isobutene or tert-butanol to give methacrolein.

2. Process according to Claim 1, wherein the regeneration stream comprises oxygen, and the catalyst regeneration phase comprises the combustion of coke deposited on the catalyst.

3. Process according to Claim 1 or 2, wherein the reaction phase has a duration of greater than or equal to 15 minutes, preferably greater than or equal to 1 hour or greater than or equal to 15 hours; and/or the regeneration phase has a duration of greater than or equal to 15 minutes and preferably greater than or equal to 5 hours.

4. Process according to one of Claims 1 to 3, wherein the reaction phase and the regeneration phase have predetermined durations; or wherein the duration of the reaction phase and/or the duration of the regeneration phase depend on the measurement of a parameter such as a temperature in the reactor or the content of a compound in a stream leaving the reactor.

5. Use of a chemical reactor comprising:

- a reaction chamber (1) containing a catalyst bed, the reaction chamber comprising a first end (2) and a second end (3) opposite the first end (2), and also a plurality of separate reaction compartments containing a catalyst bed with a system for heat exchange therebetween;
- a reaction stream supply pipe (5) connected to the inlet of the reaction chamber (1) at its first end (2);
- a reaction stream withdrawal pipe (6) connected to the outlet of the reaction chamber (1) at its second end (3);
- a regeneration stream supply pipe (8) connected to the inlet of the reaction chamber (1) at its second end (3);
- a regeneration stream withdrawal pipe (7) connected to the outlet of the reaction chamber (1) at its first end (2);
- switching means adapted to alternately allow the passage of a reaction stream in the reaction chamber (1) from the reaction stream supply pipe (5) to the reaction stream withdrawal pipe (6), and the passage of a regeneration stream from the regeneration stream supply pipe (8) to the regeneration stream withdrawal pipe (7);

to perform a chemical reaction process alternately comprising:

- a reaction phase, comprising the passage of a reaction stream in the reaction chamber (1) from its first end (2) to its second end (3); and
- a catalyst regeneration phase, comprising the passage of a regeneration stream in the reaction chamber (1) from its second end (3) to its first end (2);

the reactor preferably being a plate reactor or a multitubular reactor,
and said chemical reaction process being:

- a process for the dehydration of glycerol to give acrolein; or
- a process for the dehydration of lactic acid or ammonium lactate to give acrylic acid; or
- a process for the dehydration of 3-hydroxypropionic acid or the ammonium salt thereof to give acrylic acid; or
- a process for the dehydration of 2-hydroxyisobutyric acid or 3-hydroxyisobutyric acid or the corresponding ammonium salts thereof to give methacrylic acid; or
- a process for the conversion of a chlorinated compound into a fluorinated compound, preferably a process for the preparation of a hydrofluoroolefin or of a hydrofluorocarbon, in particular a process for the preparation of a fluoropropene and very particularly preferably a process for the preparation of 2,3,3,3-tetrafluoropropene; or
- a selective oxidation process such as the oxidation of methanol to give formaldehyde or dimethoxymethane; the oxidation of ethanol to give acetaldehyde or diethoxyethane; the oxidation of ortho-xylene or naphthalene to give phthalic anhydride; the oxidation of benzene, butene, butanol or butane to give maleic anhydride; the oxidation of propylene to give acrolein; or the oxidation of isobutene or tert-butanol to give methacrolein.

**Fig. 1**

**EP 2 858 744 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02051965 A
- US 5710356 A
- US 7763174 B
- US 20040170559 A
- DE 10239547
- US 4461745 A
- WO 2006087083 A
- EP 1848681 A

- WO 200912855 A
- WO 2009044081 A
- WO 2010046227 A
- WO 2010123154 A
- WO 2009118628 A
- US 20050228202 A
- US 5731481 A
- WO 201318915 A